# EUROPEAN PATENT APPLICATION

(11) **EP 4 563 176 A1**
(43) Date of publication of application: **04.06.2025**
(21) Application number: 23213036.9
(22) Date of filing: 29.11.2023
(51) Int. Cl.: A61M 5/34, A61M 5/32

(54) **SUPPORT ELEMENT FOR A SEPTUM OF A CARTRIDGE**

(71) Applicant: Sensile Medical AG, 4600 Olten (CH)
(72) Inventor: PFRANG, Jürgen, 93183 Kallmünz (DE); GORSHÖFER, Andreas, 93149 Nittenau (DE); FORSTER, Richard, 92269 Fensterbach (DE); SOMMER, Michael, 93051 Regensburg (DE); BERTUSCH, Isabel, 93053 Regensburg (DE)
(74) Representative: Bardehle Pagenberg Partnerschaft mbB Patentanwälte Rechtsanwälte

(57) **Abstract**

The present disclosure relates to a support element for supporting a septum of a cartridge of a medical device, in particular of a medical injection device, the support element having an axial axis and a radial axis and comprising: a first surface facing the axial axis, wherein the first surface comprises a recess configured to accommodate a septum at least partially.

## Description

### 1. Technical field

The present disclosure relates to a support element for supporting a septum of a cartridge of a medical device, in particular of a medical injection device, a needle device comprising the support element, and a medical device comprising the needle device.

### 2. Prior art

Medical devices, in particular medical injection devices, such as auto-injectors or injection pens, are known in the prior art and popular among users as they provide various benefits and can be used for various applications. Such medical devices are also sometimes referred to as drug delivery devices in particular infusion devices that are used to deliver a medicament to a user, such as a patient.

An auto-injector is a device with a pre-filled syringe, cartridge, or vial containing a medication. It is typically designed to be automated, meaning that a user may not need to manually load the medication or set the dose. Autoinjectors are often designed to be more user-friendly and may have features like automatic needle insertion and medication delivery at the push of a button. An injection pen is a device that typically comprises a cartridge or a vial containing the medication and a pen-like device with a needle at the tip. It usually requires manual assembly of the cartridge or vial into the pen and the setting of the desired dose before injection. The user typically needs to push a button or plunger to release the medication.

All of the above-mentioned types of medical devices have a liquid reservoir comprising a drug or medication, which is arranged in a containment, such as a cartridge. The cartridge typically has a sealing, such as a septum, which serves the purpose to prevent the medication from becoming contaminated. A cannula of the medical device is typically designed so as to pierce through the septum in order to provide a fluid communication from the liquid reservoir to an injection area of the user. The septum is typically an elastic part arranged at one end of the cartridge. Further, the cartridge is usually provided with a clamp or crimp to hold the septum properly in place and to connect it to the containment of the cartridge for the liquid reservoir. The cartridge and/or the liquid reservoir is typically set under pressure, for instance by a plunger for the purpose of injection to provide the drug or medication to the user.

Some medical devices only provide pressure on the liquid reservoir and/or on the cartridge upon injection. However, newer devices employ pressure on the liquid reservoir and/or on the cartridge already upon assembly of the medical device (for instance by way of a pre-loaded spring), which is advantageous for drug delivery. When the cartridge is stored under pressure in such devices for a prolonged time, the septum thereof is subjected to a pressure impact as well. This could result in damage, leading to leakage of the drug or medication within the liquid reservoir of the cartridge. Such leakage can cause an underdose (or no-dose) of the user. Typically, the processes (e.g., a filling process of the reservoir) and the components (e.g., cartridge, plunger, septum, and clamp) are quite well standardized. Thus, these parts alone do not allow for sufficient modifications.

Against this background, improvements for medical devices are needed to provide a more durable, robust, reliable, and safe medical device, which can be stored for a prolonged time without jeopardizing any leakage.

Thus, it is an object of the present disclosure to provide a support element for a needle device as used in a medical device, in particular in a medical injection device, which addresses the aforementioned needs and that overcomes the disadvantages of the present solutions at least partially. It is a further object to provide a respective needle device and a respective medical device.

### 2. Summary of the invention

The above-mentioned object is at least partially achieved by the subject-matter of the independent claims. Preferred embodiments are subject of the dependent claims, and other suitable aspects of the present invention are described through the overall disclosure of the present application.

The object is achieved by a support element for supporting a septum of a cartridge of a medical device, in particular of a medical injection device, the support element having an axial axis (AA) and a radial axis (RA) and comprising: a first surface facing the axial axis (AA), wherein the first surface comprises a recess configured to accommodate a septum at least partially.

In this manner, the present disclosure provides a support element that has the advantage of supporting a septum, such as a septum of a cartridge of a medical injection device. In particular, the support element facilitates that a pressure load to the septum can be significantly reduced. This increases robustness and durability of the septum. This may particularly be appreciated when pressure is provided on the cartridge along the axial axis. This is typically the case in various medical devices, where pressure on the cartridge and, thereby, on the septum is applied already upon assembly of the medical device. Such pressure may be provided for instance by way of a preloaded spring that applies pressure to a plunger stopper of the cartridge. Absent such a support element, the septum may be prone to damage. Accordingly, the present disclosure provides a support element to overcome this issues. In particular, the support element can facilitate a more durable septum and a more durable medical device. Thus, a more secure as well as more sterile environment can be provided for the user. This makes usage of a medical device comprising such a support element more convenient, comfortable and safer.

When the support element is employed in a needle device and/or in a medical device, the septum may be pressed against the first surface along the axial axis as understood by the skilled person. By way of the accommodation of the septum within the recess, a pressure load to the septum can be reduced. Hence, it is understood that the recess must provide for specific adaptations to allow such a reduction of pressure load to the septum. For instance, sharp edges, corners or the like of the first surface and/or the recess should be mitigated in order to support that the pressure load is reduced. The pressure load referred to herein may comprise a peak pressure. Reducing such a peak pressure has the advantage that the septum is more long-lasting. Further features of the support element to reduce the pressure load are explained in detail with regard to the preferred embodiments.

It is noted that the support element does not comprise the septum and the cartridge, however, the support element needs to be configured such that it can be used for supporting a septum of a cartridge. Thereby, it is understood that the support element needs to show features that makes it suitable for such usage. For instance, the support element must be designed in a manner that it complies with the high safety, sterility, and compatibility standards that usually pertain for such medical devices.

As derivable from the term support "element", an integral piece may be required or at least various parts that are connected to one another such that they may be regarded as one integral piece. Thereby, the term support "element" may not mean that a plurality of separate parts that are spaced apart and not connected to one another is encompassed.

The axial axis may in one example be parallel to a longitudinal axis of the needle device, when the support element is arranged in the needle device and/or in a medical device comprising such a needle device. The radial axis may be substantially perpendicular to the axial axis.

The septum, which may be supported by the support element, may provide for a sealing function. The septum may aid in maintaining the integrity and sterility of the medication within a liquid reservoir of the cartridge. The septum may be provided by any kind of suitable material including but not limited to rubber or an elastomeric material. The septum may be arranged at an end of the liquid reservoir. The septum may be able to at least partially close the piercing provided by a cannula of a needle device.

The medical device, which comprises the cartridge and the septum, may be a medical injection device. Any kind of medical injection devices are included in the present disclosure. Exemplary, the medical injection device may be an auto-injector, an injection pen, and/or a syringe. Preferably, the medical injection device is an auto-injector.

The "first surface" may be substantially perpendicular to the axial axis. This facilitates that a contact between the support septum and the septum can be established at least partially. The term "first" is not limiting and is merely used as a name for the surface.

The "recess" may be an indentation, a hole or the like. It is understood that the recess is a macroscopic recess, such that it is configured to accommodate the septum at least partially. As described elsewhere herein, the recess may be a through hole in a preferred example, however, this is not necessary. Although a cannula should be able to be guided through the recess during ordinary use of the support element, this may also be achieved when the recess is not a through hole. For instance, said cannula may be able to pierce through a part of the material of the support element.

It is noted that not the overall septum may be accommodated by the recess. Rather, a part of the septum that is in proximity to the first surface may be deformed into the recess at least partially, which contributes to a reduction of peak pressures.

It is understood that the described advantages may apply also for the following preferred embodiments.

In a preferred embodiment, the recess comprises an edge, preferably a circumferential edge, wherein the edge is provided with a radius. This facilitates that stress concentration around the edge of the recess may be mitigated. In turn, this reduces peak pressures when the septum is accommodated at least partially in the recess. This might lead to an increased strength and durability of the septum. The radius may be referred to as a rounding radius. The radius of the edge can facilitate a smoother transition, thereby reducing the chances of fracture, failure, and/or damage of the septum due to stress concentration. This effect may be pronounced in pre-pressurized medical devices. The radius may allow for easier manufacturing, and potentially easier handling due to a reduced sharpness of the edge.

In a preferred embodiment, the radius is at least 0.05 mm, preferably at least 0.10 mm, more preferably at least 0.15 mm, more preferably at least 0.2 mm, most preferably at least 0.25 mm, and/or at most 0.8 mm, preferably at most 0.7 mm, more preferably at most 0.6 mm, more preferably at most 0.5 mm, more preferably at most 0.4 mm, more preferably at most 0.3 mm, most preferably at most 0.25 mm. This further contributes to the advantages described in the foregoing embodiment. The radius should not be too large as this impairs maintaining a position of the septum. Further, the radius should not be too small, as this may lead to a sharper edge. With the radius as specified in this embodiment, an optimal balance can be struck between these different and conflicting requirements.

In a preferred embodiment, the first surface is a substantially flat surface or a conical surface with a peak of the conical surface being at an inner portion of the support element as seen along the radial axis. This has the advantage that it allows for optimal pressure distribution during ordinary use, which makes the design more robust and resilient to deformation. In addition, this has the advantage that it may be shaped in correspondence to the septum. Thereby, already the design of the first surface can contribute to a reduction of the pressure load. The conical surface can ensure stability and can distribute the pressure load throughout the surface evenly rather than concentrating it in specific areas. This can help to prevent localized wear, fatigue, or failure. Furthermore, with the peak located at the inner portion, it could make it easier to arrange the septum next to it. For instance, the septum may have a shape such that it protrudes at least partially to towards the space formed by the conical surface with the peak being at an inner portion of the support element.

In a preferred embodiment, the recess is a through hole, extending through the support element from the first surface to an opposing second surface. This leads to an enhanced functionality, easier manufacturing, and a reduced weight of the support element. In addition, the cannula of a medical device may be easily guided therethrough.

In a preferred embodiment, the recess and preferably the through hole comprise a diameter (dᵢ) as seen along the radial axis (RA) of at least 0.5 mm, preferably at least 0.8 mm, more preferably at least 1.0 mm, more preferably at least 1.5 mm, more preferably at least 1.8 mm, most preferably at least 2.0 mm, and/or of at most 5.0 mm, preferably at most 4.0 mm, more preferably at most 3.5 mm, more preferably at most 3.0 mm, more preferably at most 2.5 mm, most preferably at most 2.0 mm. Thereby, the through hole is small enough to ensure a sufficient robustness of the support element. Further, the through hole is large enough such that the septum may be accommodated.

In a preferred embodiment, the support element has an outer diameter (dₒ) as seen along the radial axis (RA) of at least 4.0 mm, preferably at least 4.5 mm, more preferably at least 5.0 mm, more preferably at least 5.5 mm, more preferably at least 6.0 mm, more preferably at least 6.5 mm, more preferably at least 7.0 mm, more preferably at least 7.5 mm, most preferably at least 7.8 mm, and/or of at most 12.0 mm, preferably at most 11.0 mm, more preferably at most 10.0 mm, more preferably at most 9.0 mm, more preferably at most 8.5 mm, most preferably at most 8.0 mm. This has the advantage that the support element is properly sized to be mounted into a needle device. Larger diameters may unnecessarily increase the amount of material that is used. Too small diameters may lead to a reduced robustness of the support element. Thus, with the outer diameter as specified in this embodiment, an optimal balance can be struck between these different and conflicting requirements. The outer diameter of the support element may correspond to the diameter of the peripheral surface of the support element as described elsewhere herein.

In a preferred embodiment, the support element has a thickness (t) as seen along the radial axis (RA) of at least 0.5 mm, preferably at least 0.8 mm, more preferably at least 1.0 mm, more preferably at least 1.5 mm, more preferably at least 1.8 mm, more preferably at least 2.0 mm, more preferably at least 2.3 mm, most preferably at least 2.5 mm, and/or of at most 7.0 mm, preferably at most 6.0 mm, more preferably at most 5.0 mm, preferably at most 4.0 mm, more preferably at most 3.5 mm, more preferably at most 3.0 mm, more preferably at most 2.8 mm, most preferably at most 2.5 mm. This further contributes to the advantages set out elsewhere herein. The thickness should not be too large as this makes the support element bulky and requires more material. Further, the thickness should not be too small, as this may prevent that the septum can be accommodated. With the thickness as specified in this embodiment, an optimal balance can be struck between these different and conflicting requirements.

In a preferred embodiment, the support element further comprises a peripheral surface comprising a mounting portion configured such that the support element can be mounted inside a hub portion of a part of the medical device, preferably by press-fitting. The mounting portion may be implemented as a flattened surface. The mounting portion may have the advantage to prevent rotation of the support element, when mounted. Mounting may be performed by press-fitting. Thereby, a force along the radial axis may be provided leading to a substantially even pressure distribution on the mounting portion. Additionally, or alternatively mounting means such as adhesives, fasteners or the like may be employed. The part of the medical device may be a needle device as described elsewhere herein.

In a preferred embodiment, the mounting portion has a length (1) compared to an outer diameter (dₒ) of the support element of at least 10%, preferably at least 20%, more preferably at least 25%, most preferably at least 30%, and/or of at most 70%, preferably at most 60%, more preferably at most 50%, most preferably at most 40%. The length of the mounting portion may be measured substantially perpendicular to the axial axis. The values given here for the length of the mounting portion allow better mounting.

In a preferred embodiment, the support element comprises a solid material, preferably one or more of the following: metal, ceramic, glass, polymer, in particular polycarbonate. A solid material facilitates robustness of the support element. The materials mentioned in this embodiment can be easily procured and can impart specific properties to the support element. Metals offer high strength, durability, and resistance to wear and tear. Ceramics are hard, strong, and often resistant to wear, heat and chemicals. Polymers are lightweight materials with good flexibility and strength. Polycarbonate is a type of polymer and has a high impact resistance, transparency, and stability under a wide temperature range.

The object of the present disclosure is also achieved by a needle device for a medical device, in particular for a medical injection device, the device comprising: a hub portion defining a longitudinal axis and having a cavity for accommodating a cartridge comprising a septum at least partially; a support element as described in here, wherein the support element is arranged in the cavity and mounted to the hub portion, preferably by press-fitting. As understood, since the needle device comprises the support element as described elsewhere herein, it is understood that the features and/or advantages described with reference to the support element may also apply for the needle device and vice versa. The support element may be provided slightly oversized compared to the hub portion in which it is arranged. This may aid in providing an improved and secured fit. The needle device could be a needle safety device.

The object of the present disclosure is also achieved by a cartridge for a medical device, in particular for a medical injection device, the cartridge comprising: a septum; a support element as described in here, the support element being arranged in proximity to the septum; and optionally, a crimp or clamp, wherein the crimp or clamp preferably at least partially accommodates the septum and, distally thereto, the support element. As understood, since the cartridge comprises the support element as described elsewhere herein, the features and/or advantages described with reference to the support element may also apply for the cartridge and vice versa. The support element is accommodated distally with respect to the septum. This means that the support element is arranged more to a distal end of the cartridge along the axis from the proximal end to the distal end of the cartridge compared to the septum.

In a preferred embodiment, the cavity comprises a first cavity and a second cavity, wherein the support element is arranged in the first cavity and the second cavity is configured to accommodate a cartridge at least partially, wherein the hub portion comprises a projection extending at least partially into the first cavity so as to provide a pressure contact with the mounting portion of the support element.

By having a first cavity and second cavity, any components arranged therein may be kept segregated in one example. This may simplify the assembly process. The support element arranged in the first cavity and the cartridge in the second cavity allows for a structured arrangement of the respective components. The projection may be a structure that protrudes from the hub portion of the needle device. The projection may be integrally formed with the hub portion of the needle device, or it may be provided as a separate part. The projection has the advantage of improving mounting of the support element in the hub portion. The projection provides the benefit of a secured, and precise fit. This can enhance the stability and reliability of the connection, mitigating the risk of loosening, misalignment or disassembly.

The first cavity and the second cavity may be in fluid communication with one another. The first cavity and the second cavity may be substantially in proximity to one another. The first cavity and the second cavity may be directly adjacent to one another.

In a preferred embodiment, the hub portion comprises an abutting surface facing the longitudinal axis so as to provide a pressure contact with the support element, preferably with at least a part of the second surface of the support element. The second surface of the support element may be the surface opposite to the first surface of the support element as described elsewhere herein. The benefit of the abutting surface may be to ensure stability, reliability, and maintaining the position of the support element. The pressure contact generated by the abutting surface against the support element can provide a secure and stable fit. The application of the pressure evenly across the abutting surface as opposed to concentrated points can help distribute stresses, potentially reducing wear and tear, and prolonging the lifespan. The described configuration of this embodiment may also aid in precise alignment and positioning of the components. The abutting surface may be substantially flat. The abutting surface may have an annular shape. For instance, the abutting surface may define an opening, such as an opening through which a cannula can be guided.

The object of the present disclosure is also achieved by a medical device, in particular a medical injection device, comprising a needle device as described herein and a cartridge having a septum arranged preferably in a crimp or clamp of the cartridge. As understood, since the medical device comprises the needle device as described elsewhere herein, it is understood that the features and/or advantages described with reference to the needle device and/or the support element may also apply for the medical device and vice versa. The crimp or clamp may house the septum at least partially. The crimp or clamp may come in contact with the first surface of the support element at least partially, when the medical device is set under pressure. A crimp may be understood as any means suitable for holding, fixing, and/or maintaining a part, such as the septum in this particular case. A crimp may be deformed at least partially so as to hold the septum. A clamp may be any means suitable for at least partially clamping a part, such as the septum in this particular case.

In a preferred embodiment, the recess of the first surface of the support element is configured such that the septum is at least partially accommodated therein when the septum is pressed towards the first surface along the axial axis. As described elsewhere herein, said pressing may be provided by a spring or the like. In one example, the septum may be deformed into the recess at least partially due to the pressure.

In a preferred embodiment, the first surface is shaped substantially in correspondence to the shape of a septum and/or a crimp or clamp of the cartridge which is pressed towards the support element. For instance, the first surface of the support element may be a flat surface and the surface of the septum in proximity thereto may also be a flat surface. Further, the surface of the septum may be angled with a protrusion towards the longitudinal axis at a radially central position of the septum. This may help to support the septum in an improved manner.

In a preferred embodiment, the medical injection device is an auto-injector or an injection pen.

### 4. Brief description of the accompanying figures

In the following, the invention will be described in more detail with reference to the following figures:
- **Fig. 1:**: shows a support element according to an embodiment of the present invention.
- **Fig. 2:**: shows a part of a needle device according to an embodiment of the present invention in a cross-sectional cut.
- **Fig. 3:**: shows a part of a medical device according to an embodiment of the present invention in a cross-sectional cut.
- **Fig. 4:**: shows a part of a medical device according to an embodiment of the present invention in a cross-sectional cut.
- **Fig. 5:**: shows a medical device according to an embodiment of the present invention.

### 5. Detailed description of the figures

In the following only some possible embodiments of the invention are described in detail. However, the present invention is not limited to these, and a multitude of other embodiments are applicable without departing from the scope of the invention. The presented embodiments can be modified in a number of ways and combined with each other whenever compatible and certain features may be omitted in so far as they appear dispensable. In particular, the disclosed embodiments may be modified by combining certain features of one embodiment with one or more features of another embodiment.

Throughout the present figures and specification, the same reference numerals refer to the same elements. For the sake of clarity and conciseness, certain aspects of components or steps of certain embodiments are presented without undue detail where such detail would be apparent to those skilled in the art in light of the teachings herein and/or where such detail would obfuscate an understanding of more pertinent aspects of the embodiments.

### Definitions

The "proximal" end as used herein is merely used to illustrate one of two ends of a part. The proximal end of a part may be understood best in combination with a distal end of the same part. The terms proximal and distal are not to be understood limiting by any means. The ends could additionally or alternatively be referred to as first and second end or second and first end, respectively.

The distal end of a part may usually be an end that is further away from a center point of the medical device compared to the proximal end of the part. Further, the distal end of a part may usually be an end that is closer to an injection area compared to the proximal end of the part when the medical device is used according to its ordinary use.

Same applies to the terms "proximal direction" and "distal direction". That is, these terms could additionally or alternatively be referred to as first and second direction or second and first direction. The proximal direction may be a direction from a distal end to a proximal end. The distal direction may be a direction from a proximal end to a distal end.

Unless otherwise stated, the term "substantial" or "substantially" as used in the present context may be understood to a great or significant extent or for the most part or essentially. In particular, manufacturing tolerances are included by this term.

### Description of preferred embodiments

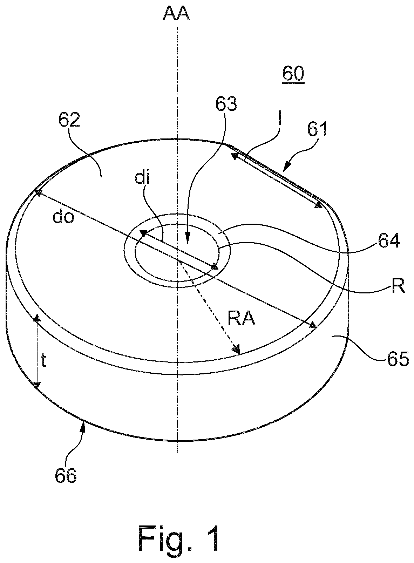

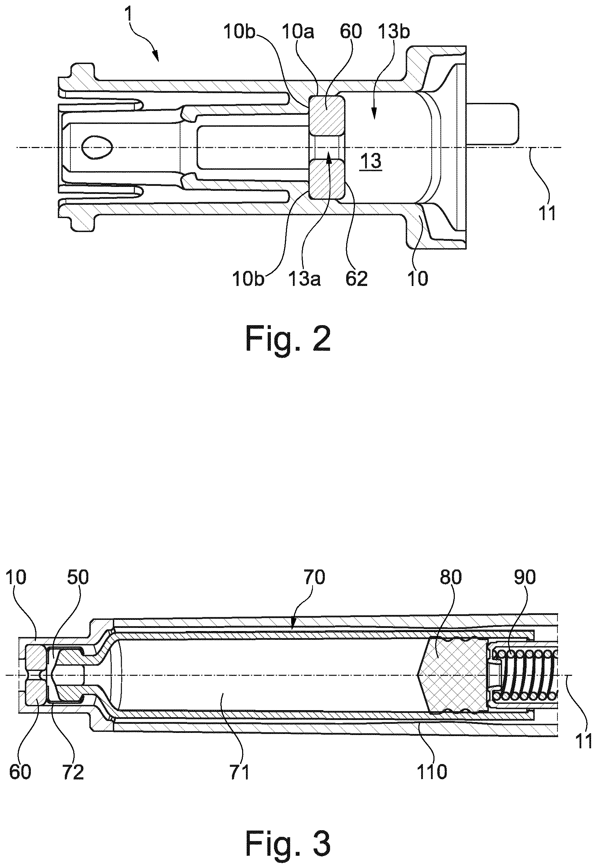

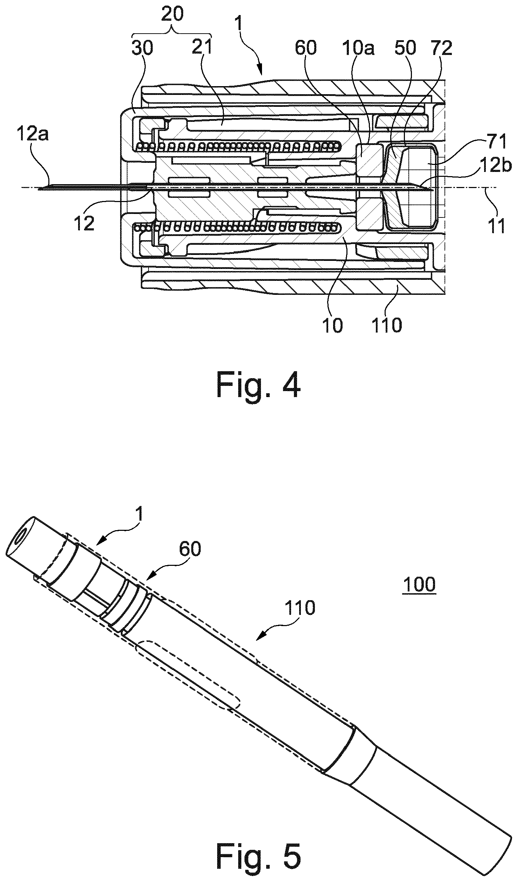

**Fig. 1** shows a support element 60 for supporting a septum 50 (as seen in Fig. 3, Fig. 4) of a cartridge 70 (as seen in Fig. 3) of a medical device 100, in particular of a medical injection device. The support element 60 has an axial axis AA and a radial axis RA and comprises a first surface 62 facing the axial axis AA.

The first surface 62 comprises a recess 63 configured to accommodate a septum 50 at least partially (as seen in Fig. 3). The recess 63 comprises an edge 64, preferably a circumferential edge 64, wherein the edge 64 is provided with a radius R. The radius R can be at least 0.05 mm, and/or at most 0.8 mm as described elsewhere herein. The first surface 62 is a substantially flat surface or a conical surface with a peak of the conical surface being at an inner portion of the support element 60 as seen along the radial axis RA. The inner portion could be for instance at halfway of the thickness t of the support element 60 along the axial axis AA. Further, the inner portion could be at a distance of at least 10%, 20%, 30%, 40%, 50%, 60%, 70%, 80%, 90% of the thickness t of the support element 60 along the axial axis AA.

As can be seen, the recess 63 is a through hole, extending through the support element 60 from the first surface 62 to an opposing second surface 66. However, the recess 63 does not necessarily have to be a through hole. The recess 63, in particular the through hole, comprises a diameter dᵢ as seen along the radial axis RA of at least 0.5 mm and/or of at most 5.0 mm, as described elsewhere herein.

The support element 60 has an outer diameter dₒ as seen along the radial axis RA of at least 4.0 mm and/or of at most 12.0 mm, as described elsewhere herein. The outer diameter dₒ of the support element 60 may correspond to the diameter of the peripheral surface 65 of the support element 60. The support element 60 has a thickness t as seen along the radial axis RA of at least 0.5 mm and/or of at most 5.0 mm, as described elsewhere herein.

The support element 60 further comprises a peripheral surface 65 comprising a mounting portion 61 configured such that the support element 60 can be mounted inside a hub portion 10 (as seen in Fig. 2) of a part of the medical device 100, preferably by press-fitting. The mounting portion 61 has a length l compared to an outer diameter d₀ of the support element 60 of at least 10% and/or of at most 70%, as described elsewhere herein.

The support element 60 can comprise a solid material, preferably one or more of the following: metal, ceramic, glass, polymer, in particular polycarbonate.

As can be seen in Fig. 1, the support element 60 may be an annular support element 60, such as a ring, comprising a recess 63, in particular a through hole. This through hole may enable a cannula 12 to be guided therethrough so as to puncture a septum 50 (as seen in Fig. 4).

**Fig. 2** shows a part of a needle device 1 according to an embodiment of the present invention in a cross-sectional cut. The needle device 1 is for a medical device 100, in particular for a medical injection device. The needle device 1 comprises a hub portion 10 defining a longitudinal axis 11 and having a cavity 13 for accommodating at least partially a cartridge 70 comprising a septum 50. The needle device 1 also comprises a support element 60 as described in here, wherein the support element 60 is arranged in the cavity 13 and mounted to the hub portion 10, preferably by press-fitting. The cavity 13 comprises a first cavity 13a and a second cavity 13b, wherein the support element 60 is arranged in the first cavity 13a and the second cavity 13b is configured to accommodate a cartridge 70 at least partially. The hub portion 10 comprises a projection 10a extending at least partially into the first cavity 13a so as to provide a pressure contact with the mounting portion 61 of the support element 60.

The hub portion 10 comprises an abutting surface 10b facing the longitudinal axis 11 so as to provide a pressure contact with the support element 60, preferably with at least a part of the second surface 66 (as seen in Fig. 1) of the support element 60. The second surface 66 of the support element 60 may be the surface opposite to the first surface 62 of the support element 60 as described elsewhere herein.

The septum 50 may prevent air, contaminants, and moisture from entering the liquid reservoir 71 (as seen in Fig. 3) and affecting the stability and efficacy of the medication. Typically, when a medical device 100 is activated, the cannula 12 pierces or punctures the septum 50 to access the medication. This piercing or puncture facilitates the medication to be drawn out of the liquid reservoir 71. The septum 50 may ensure that the medication remains sterile until the moment of administration.

**Fig. 3** shows a part of a medical device 100 according to an embodiment of the present invention in a cross-sectional cut. The medical device comprises a needle device 1 as described in here and a cartridge 70 having a septum 50 arranged preferably in a crimp 72 of the cartridge 70. The cartridge 70 comprises a liquid reservoir 71. As can be derived from Fig. 3 and from Fig. 4, the recess 63 of the support element 60 is configured such that the septum 50 is at least partially accommodated therein when the septum 50 is pressed towards the first surface 62 along the axial axis AA. Such pressing may be provided by way of the biasing element 90 for the plunger 80. By virtue of the biasing element 90, the cartridge 70 is pushed against the support element 60, thereby contacting the first surface 62 of the support element 60 with the overpressure side of the septum (the one facing the left-hand side in Fig. 3). Medical devices 100 where such a pressure is provided already subsequent to assembly may be referred to as pre-pressurized medical devices 100, in particular pre-pressurized medical injection devices 100.

The liquid reservoir 71 may be within a cartridge 70, a barrel or the like. In various examples, the liquid reservoir 71 may be housed within a cylindrical glass or plastic container forming part of the cartridge 70. The liquid reservoir 71 may comprise a medication or a component thereof. It is noted that the liquid reservoir 71 could alternatively or additionally be located further away from the septum 50. The fluid communication from the liquid reservoir 71 to an injection area of the user by way of the cannula 12 merely serves the purpose that medication can be delivered. This may be independent on whether or not the liquid reservoir 71 is located directly adjacent to the septum 50.

Further, the first surface 62 can be shaped substantially in correspondence to the shape of the septum 50 and/or the crimp 72 of the cartridge 70 which is pressed towards the support element 60.

The medical injection device 100 depicted in here and described throughout the present disclosure can be an auto-injector or an injection pen.

**Fig. 4** shows more details of a part of a medical device 100 according to an embodiment of the present invention in a cross-sectional cut. In particular, details of a needle device 1 are shown. The needle device 1 could be a needle safety device.

Some general features of the needle device 1 may be described to illustrate an understanding of the present disclosure. The needle device 1 comprises a hub portion 10 defining a longitudinal axis 11. The hub portion 10 comprises a cannula 12 extending from the hub portion 10 substantially along the longitudinal axis 11. The cannula 12 may be configured to be moved along the longitudinal axis 11 relative to the hub portion 10. The needle device 1 also comprises a tubular arrangement 20 being movably arranged on the hub portion 10 to move along the longitudinal axis 11 relative to the hub portion 10. The tubular arrangement 20 is configured to be moved between an initial position and an injection position (the latter one is depicted in Fig. 4). In the injection position, a proximal end 12b of the cannula 12 is configured to protrude through the septum 50 of the medical device 100 such that the cannula 12 is in fluid communication with the liquid reservoir 71 of the cartridge 70 (indicated in Fig. 3) of the medical device 100. The distal end 12a of the cannula 12 is opposite the proximal end 12b and is configured to come in contact with an injection site or injection are of a user.

The term cannula 12 as used in the present disclosure may additionally or alternatively be referred to as a needle or injection needle. The cannula 12 may be hollow, so as to guide a fluid therethrough. Further, the cannula 11 may be attached directly or indirectly to the hub portion 10. The cannula 12 may be part of a cannula arrangement. For instance, the cannula arrangement may comprise a plurality of cannulas. The plurality of cannulas may be connected to one another, for instance along one direction, in particular along the longitudinal axis of the hub portion 10. The plurality of cannulas may partially overlap one another along the longitudinal axis, which may serve the purpose to fix the cannula arrangement.

Although not shown in the figures, it is noted that the support element 50 may also be arranged at different locations of the medical device. One embodiment is directed to a cartridge 70 for a medical device, in particular for a medical injection device 100. The cartridge 70 comprises a septum 50, a support element 60 as described in here and optionally a crimp 72 or clamp. The support element 60 may be arranged in proximity to the septum 50. Preferably, the crimp 72 or clamp at least partially accommodates the septum 50 and, distally thereto, the support element 60.

**Fig. 5** shows a medical device 100 according to an embodiment of the present invention. The medical device 100 can be a medical injection device 100 and comprises the needle device 1 as described above. The medical injection device 100 comprises a housing 110 which engages with a part of the needle device 1. Moreover, the septum 50 is indicated. However, as the septum 50 is arranged within the medical device 100, it cannot be seen from outside as understood by the skilled person.

In the following, some general features of the needle device 1 of the present disclosure are explained in greater detail, in particular when the needle device 1 is a needle safety device 1.

The tubular arrangement 20 may comprise a first tubular element 21 and a second tubular element 30. The first tubular element 21 may be movably arranged on the hub portion 10 to move along the longitudinal axis 11. The first tubular element 21 may comprise a guide track which slidably engages with a guide pin of the hub portion 10. The guide track may be configured such that the first tubular element 21 rotates relative to the hub portion 10 when the guide pin moves along the guide track, wherein the guide track may comprise a final location configured to retain the guide pin. The second tubular element 30 may be arranged on the first tubular element 21 such that the second tubular element 30 is movable along the longitudinal axis 11 together with the first tubular element 21. The first tubular element 21 may be at least partially enclosed by the second tubular element 30. The second tubular element 30 may comprise a contact surface 31 for establishing pressure contact with an injection area.

It is noted that any one or more of the embodiments described herein and/or examples may be combined with further aspects as described herein and details of the embodiments and/or examples may also be omitted, as will be understood by the skilled person. The scope of protection is determined by the claims and is not limited by the embodiments and/or examples disclosed in the above figures.

### List of reference signs

- 1: needle safety device
- 10: hub portion
- 10a: projection (abutting surface in radial direction)
- 10b: abutting surface in axial direction
- 11: longitudinal axis
- 12: cannula
- 12a: distal end of the cannula
- 12b: proximal end of the cannula
- 13: cavity
- 13a: first cavity (for septum)
- 13b: second cavity (for at least a part of the cartridge)
- 20: tubular arrangement
- 21: first tubular element
- 30: second tubular element
- 50: septum
- 60: support element for a septum
- 61: mounting portion of support element
- 62: first surface facing axial axis
- 63: recess of support element
- 64: circumferential edge
- 65: peripheral surface
- 66: second surface facing axial axis (opposite to first surface)
- 70: cartridge
- 71: liquid reservoir
- 72: crimp
- 80: plunger
- 90: biasing element for the plunger
- 100: medical injection device
- 110: housing

- AA: axial axis of the support element
- RA: radial axis of the support element
- R: Radius of the circumferential edge
- dᵢ: diameter of the recess (inner diameter of support element)
- dₒ: outer diameter of support element (outer diameter of peripheral surface)
- l: length of mounting portion
- t: thickness of the support element

## Claims

1. A support element (60) for supporting a septum (50) of a cartridge (70) of a medical device, in particular of a medical injection device (100), the support element (60) having an axial axis (AA) and a radial axis (RA) and comprising:
a first surface (62) facing the axial axis (AA), wherein the first surface (62) comprises a recess (63) configured to accommodate a septum (50) at least partially.

2. The support element (60) for a septum (50) according to the preceding claim, wherein the recess (63) comprises an edge (64), preferably a circumferential edge, wherein the edge (64) is provided with a radius (R).

3. The support element (60) for a septum (50) according to the preceding claim, wherein the radius (R) is at least 0.05 mm, preferably at least 0.10 mm, more preferably at least 0.15 mm, more preferably at least 0.2 mm, most preferably at least 0.25 mm, and/or
at most 0.8 mm, preferably at most 0.7 mm, more preferably at most 0.6 mm, more preferably at most 0.5 mm, more preferably at most 0.4 mm, more preferably at most 0.3 mm, most preferably at most 0.25 mm.

4. The support element (60) for a septum (50) according to any one of the preceding claims, wherein the first surface (62) is a substantially flat surface or a conical surface with a peak of the conical surface being at an inner portion of the support element (60) as seen along the radial axis (RA).

5. The support element (60) for a septum (50) according to any one of the preceding claims, wherein the recess (63) is a through hole, extending through the support element (60) from the first surface (62) to an opposing second surface.

6. The support element (60) for a septum (50) according to any one of the preceding claims, wherein the recess (63) and preferably the through hole comprise a diameter (dᵢ) as seen along the radial axis (RA) of at least 0.5 mm, preferably at least 0.8 mm, more preferably at least 1.0 mm, more preferably at least 1.5 mm, more preferably at least 1.8 mm, most preferably at least 2.0 mm, and/or
of at most 5.0 mm, preferably at most 4.0 mm, more preferably at most 3.5 mm, more preferably at most 3.0 mm, more preferably at most 2.5 mm, most preferably at most 2.0 mm.

7. The support element (60) for a septum (50) according to any one of the preceding claims, wherein the support element (60) has an outer diameter (dₒ) as seen along the radial axis (RA) of at least 4.0 mm, preferably at least 4.5 mm, more preferably at least 5.0 mm, more preferably at least 5.5 mm, more preferably at least 6.0 mm, more preferably at least 6.5 mm, more preferably at least 7.0 mm, more preferably at least 7.5 mm, most preferably at least 7.8 mm, and/or
of at most 12.0 mm, preferably at most 11.0 mm, more preferably at most 10.0 mm, more preferably at most 9.0 mm, more preferably at most 8.5 mm, most preferably at most 8.0 mm.

8. The support element (60) for a septum (50) according to any one of the preceding claims, wherein the support element has a thickness (t) as seen along the axial axis (RA) of at least 0.5 mm, preferably at least 0.8 mm, more preferably at least 1.0 mm, more preferably at least 1.5 mm, more preferably at least 1.8 mm, more preferably at least 2.0 mm, more preferably at least 2.3 mm, most preferably at least 2.5 mm, and/or
of at most 7.0 mm, preferably at most 6.0 mm, more preferably at most 5.0 mm, more preferably at most 4.0 mm, more preferably at most 3.5 mm, more preferably at most 3.0 mm, more preferably at most 2.8 mm, most preferably at most 2.5 mm.

9. The support element (60) for a septum (50) according to any one of the preceding claims, further comprising a peripheral surface (65) comprising a mounting portion (61) configured such that the support element can be mounted inside a hub portion (10) of a part of the medical device, preferably by press-fitting.

10. The support element (60) for a septum (50) according to the preceding claim, wherein the mounting portion (61) has a length (l) compared to an outer diameter (dₒ) of the support element (60) of at least 10%, preferably at least 20%, more preferably at least 25%, most preferably at least 30%, and/or
of at most 70%, preferably at most 60%, more preferably at most 50%, most preferably at most 40%.

11. The support element (60) for a septum (50) according to any one of the preceding claims, wherein the support element (60) comprises a solid material, preferably one or more of the following: metal, ceramic, glass, polymer, in particular polycarbonate.

12. A needle device (1) for a medical device, in particular for a medical injection device (100), the needle device (1) comprising:
a hub portion (10) defining a longitudinal axis (11) and having a cavity (13) for accommodating a cartridge (70) comprising a septum (50) at least partially;
a support element (60) according to any one of the proceeding claims;
wherein the support element (60) is arranged in the cavity (13) and mounted to the hub portion (10), preferably by press-fitting.

13. The needle device (1) according to the preceding claim, wherein the cavity (13) comprises a first cavity (13a) and a second cavity (13b), wherein the support element (60) is arranged in the first cavity (13a) and the second cavity (13b) is configured to accommodate a cartridge (70) at least partially,
wherein the hub portion (10) comprises a projection (10a) extending at least partially into the first cavity (13a) so as to provide a pressure contact with the mounting portion (61) of the support element (60).

14. A cartridge (70) for a medical device, in particular for a medical injection device (100), the cartridge (70) comprising:
a septum (50);
a support element (60) according to any one of claims 1 to 11, the support element (60) being arranged in proximity to the septum (50); and
optionally a crimp (72) or clamp, wherein the crimp (72) or clamp preferably at least partially accommodates the septum (72) and, distally thereto, the support element (60).

15. A medical device, in particular a medical injection device (100), comprising a needle device (1) according to any one of the preceding claims 12 or 13 and a cartridge (70) having a septum (50) arranged preferably in a crimp (72) or clamp of the cartridge (70),
wherein the medical device is preferably an auto-injector or an injection pen.
